# EUROPÄISCHE PATENTSCHRIFT

(11) **EP 3 724 316 B1**
(45) Veröffentlichungstag und Bekanntmachung des Hinweises auf die Patenterteilung: **03.05.2023**
(21) Anmeldenummer: 17828879.1
(22) Anmeldetag: 15.12.2017
(51) Int. Cl.: C12M 1/32, C12M 1/12, C12M 1/00

(54) **ZELLKULTURTRÄGER**
CELL CULTURE CARRIER
SUBSTRAT DE CULTURE CELLULAIRE

(43) Veröffentlichungstag der Anmeldung: 21.10.2020
(73) Patentinhaber: Technische Universität Ilmenau, 98693 Ilmenau (DE)
(72) Erfinder: SCHOBER, Andreas, 99096 Erfurt (DE); WEISE, Frank, 98693 Ilmenau (DE); HAMPL, Jörg, 99090 Erfurt (DE)
(74) Vertreter: Engel, Christoph Klaus
(86) Internationale Anmeldenummer: PCT/EP2017/083151
(87) Internationale Veröffentlichungsnummer: WO 2019/114997

(56) Entgegenhaltungen:
- WO-A2-2009/121868
- DE-U1-202011 003 049
- US-A1- 2005 101 010
- US-A1- 2007 141 555
- US-A1- 2008 076 170
- US-A1- 2010 190 197
- US-A1- 2017 218 321
- US-B1- 6 794 184

## Beschreibung

Die Erfindung betrifft einen Zellkulturträger zur Kultivierung von biologischem Zellmaterial. Der Zellkulturträger besitzt eine Trägerplatte, die bevorzugt an externe Einheiten angepasst ist, in denen zumeist eine Vielzahl solcher Zellkulturträger zusammengefasst und mit den für die Zellkultivierung erforderlichen Medien versorgt werden. Der Zellkulturträger besitzt außerdem eine poröse Membran, die von der Trägerplatte getragen wird und bevorzugt als strukturierter Formkörper ausgebildet ist. Die Membran ist für einen Hauptfluss einer Nährstofflösung durchlässig und stellt eine Ansiedlungsfläche für das zu kultivierende Zellmaterial bereit. Weiterhin umfasst der Zellkulturträger einen Aufnahmekäfig oder eine vergleichbare Kavität, in welchen das Zellmaterial einbringbar ist. Der Aufnahmekäfig ist auf der Membran angeordnet, in diese eingesetzt oder einstückig mit dieser gebildet, sodass eine Stirnseite des Aufnahmekäfigs durch die Membran abgedeckt ist, um da Zellmaterial im Käfig zu halten. Der Aufnahmekäfig wird im Betrieb vom Hauptfluss durchströmt, um das Zellmaterial mit Nährstoffen, Sauerstoff oder dergleichen zu versorgen.

Biologisches Zellmaterial, also beispielsweise Gewebestücke, Biopsiematerial oder multizelluläre gewebeähnliche Zellverbände müssen in lebensförderlichen Bedingungen kultiviert werden, wenn sie außerhalb ihres natürlichen Umfeldes über einen mittelfristigen Zeitraum von mehreren Stunden bis zu einigen Wochen untersucht und ggf. vermehrt werden sollen. Dazu werden sie auf Zellkulturträgern abgelegt und dort mit den passenden Medien bzw. Nährstoffen versorgt, wobei ein Fluid das Zellmaterial perfundiert, um Nährstoffe und gelösten Sauerstoff an die einzelnen Zellen heranzuführen. Im einfachsten Fall werden die Zellmaterialien in den Wells von Mikrotiterplatten abgelegt, welche mit besonderen Einsätzen ausgerüstet sein können.

Die US 2007/0141555 A1 zeigt eine Vorrichtung zum Beobachten von Zellen. Die Vorrichtung umfasst einen Strömungsdämpfer mit einer Dämpfungsfläche, in Form einer porösen Membran, und einer kreisförmigen Außenwand. Der Strömungsdämpfer ist in einem Behälter, der beispielsweise durch eine Mikrotiterplatte gebildet werden kann, angeordnet. Der Behälter weist auf einer unteren Bodenfläche Picowells auf, in welchen Zellen separiert angeordnet werden können.

Aus der US 6 794 184 B1 ist eine Zellkulturvorrichtung bekannt, welche eine Vielzahl Kulturbehälter zur Aufnahme eines Kulturmediums sowie poröse Zellkultureinsätze mit einer Zellkulturoberfläche aufweist. Je ein Zellkultureinsatz ist in je einen Kulturbehälter beweglich einsetzbar.

In der DE 20 2006 017 853 U1 ist ein Einsatz für eine Mikrotiterplatte beschrieben, bestehend aus einer Trägerstruktur, in die mindestens eine Vertiefung eingebracht ist, wobei der obere Durchmesser der Vertiefung so gewählt ist, dass sich diese in eine Vertiefung der Mikrotiterplatte einsetzen lässt. Der Boden der Vertiefung besitzt jeweils mindestens eine nach unten ausgeformte Mikrokavität. Der Einsatz ist zumindest teilweise mit Poren versehen.

Neben Mikrotiterplatten gibt es auch andere Ansätze, um Kavitäten für die Kultivierung und Untersuchung biologischer Substanzen bereitzustellen.

So beschreibt die WO 2011/035937 A1 einen mikrostrukturierten Formkörper, umfassend eine Folie, die in unverformte Bereiche und verdünnte Verstreckungsbereiche geteilt ist. Zumindest in einigen der verdünnten Verstreckungsbereiche sind Mikrostrukturen ausgebildet, wobei Poren zumindest in einem der verdünnten Verstreckungsbereiche ausgebildet sind und zumindest einige der unverformten Bereiche undurchlässig sind.

Weiterhin ist aus der WO 2011/035938 A1 ein mikrostrukturierter Formkörper bekannt, der einen folienartigen Grundkörper besitzt, welcher eine erste Folienschicht und eine darunter befindliche zweite Folienschicht umfasst, wobei die zweite Folienschicht Ausnehmungen mit einem Durchmesser von weniger als 2 mm aufweist, die durch verformte Bereiche der ersten Folienschicht ausgeformt sind, durch welche Kavitäten ausgebildet sind. Zumindest einige der verformten Bereiche der ersten Folienschicht besitzen Poren. Die Bereiche des folienartigen Grundkörpers sind außerhalb der Ausnehmungen undurchlässig.

In der DE 10 2010 037 968 A1 ist eine Struktur zur Nachbildung eines Sinusoids beschrieben, die sich in eine Mikrotiterplatte einsetzen lässt. Die Struktur umfasst mehrere übereinander angeordnete Schichten aus einem porösen Material, wobei zwischen den Schichten jeweils ein Zwischenraum ausgebildet ist. Die Zwischenräume sind durch in den Schichten zur Weiterleitung eines Fluids ausgebildete Kanäle verbunden.

Es hat sich jedoch gezeigt, dass für die Versorgung bestimmter Zellmaterialien eine hohe Transportrate der Nährstoffe (einschließlich gelöste Gase, insbesondere Sauerstoff) erforderlich ist. Am einfachsten lässt sich dies durch hohe Fließgeschwindigkeiten des die Nährstoffe beinhaltenden Mediums erreichen. Allerdings führt eine hohe Fließgeschwindigkeit zu hohen Scherkräften in den Zellverbänden, denen empfindliche Gewebebereiche nicht widerstehen können. Dies führt letztlich zur Zerstörung des Zellmaterials. Im Stand der Technik sind Versuche bekannt geworden, die scherkraftbedingte Auflösung eines Zellverbandes durch Einschließen in einen Aufnahmekäfig zu verhindern, wodurch sich das geschilderte Problem aber nicht lösen lässt. Entweder behindert ein eng geschlossener Käfig die gewünschte hohe Transportrate oder ein weit geöffneter Käfig kann das Zellmaterial nicht hinreichend schützen.

Eine Aufgabe der Erfindung besteht ausgehend von diesem Problem darin, einen verbesserten Zellkulturträger bereit zu stellen, mit Hilfe dessen biologisches Zellmaterial mittelfristig kultiviert werden kann, wobei sich die Transportrate der benötigten Nährstoffe deutlich erhöhen lassen soll, ohne dass erhöhte Fließgeschwindigkeiten der Nährstofflösung zu einer Auflösung oder Zerstörung der kultivierten Zellverbände führt.

Diese Aufgabe wird durch einen Zellkulturträger gemäß dem beigefügten Anspruch 1 sowie eine Zellkulturträgeranordnung gemäß Anspruch 10 gelöst.

Der erfindungsgemäße Zellkulturträger zeichnet sich dadurch aus, dass die Trägerplatte mehrere Strömungsöffnungen bereitstellt, die umfangseitig verteilt außerhalb des Aufnahmekäfigs positioniert sind. Die mehreren Strömungsöffnungen gestatten einen Nebenfluss der Nährstofflösung, dessen Fließgeschwindigkeit größer ist als die Fließgeschwindigkeit des durch den Aufnahmekäfig und die Ansiedlungsfläche strömenden Hauptflusses der Nährstofflösung.

Der Aufnahmekäfig stellt seinerseits einen porösen Behälter für das Zellmaterial bereit. Bevorzugt besteht die Wandung des Aufnahmekäfigs vollständig aus porösem Material, welches ein Durchströmen der Nährstofflösung gestattet. Vorzugsweise sind die Stirnseiten des Aufnahmekäfigs komplett geöffnet, wobei die untere Stirnseite durch die Membran geschlossen wird, um das Zellmaterial im Aufnahmekäfig zu halten. Die obere Stirnseite kann hingegen offen bleiben, um das Einsetzen des Zellmaterials zu ermöglichen und ggf. auch optische Beobachtungen zu gestatten. Der Hauptfluss der Nährstofflösung durchströmt den Aufnahmekäfig axial und stellt die Grundversorgung der Zellen sicher. Darüber hinaus wird an der Außenseite des Aufnahmekäfigs der Nebenfluss entlang geführt, sodass über die porösen Seitenwände des Aufnahmekäfigs ebenfalls ein Nährstofftransport erfolgen kann. Aufgrund der höheren Fließgeschwindigkeit des Nebenflusses im Vergleich zum Hauptfluss ist die Transportrate für die Nährstoffe hoch, denn es entsteht ein großer Gradient an Nährstoffen, Sauerstoff und Zellabbauprodukten zwischen dem Inneren des Aufnahmekäfigs und der vom Nebenfluss versorgten Außenseite, was trotz der Barrierewirkung des Käfigs eine verbesserte Versorgung der kultivierten Zellen ermöglicht. Innerhalb des Aufnahmekäfigs bleibt das Zellmaterial geschützt und es herrscht eine geringere Fließgeschwindigkeit.

Gemäß einer vorteilhaften Ausführungsform ist der Aufnahmekäfig aus demselben porösen Material wie die Membran gefertigt, beispielsweise aus Polycarbonat. Auch die Porengröße kann in der Wandung des Aufnahmekäfigs identisch zur Membran gewählt sein, was zu günstigen dynamischen Druckverhältnissen innerhalb des Aufnahmekäfigs führt.

Bevorzugt ist der Aufnahmekäfig als Hülse mit offenen Stirnseiten geformt, insbesondere mit zylindrischer Form. Es können aber auch rechteckige oder polygonale Querschnitte für den Aufnahmekäfig gewählt werden.

Eine bevorzugte Ausführungsform zeichnet sich dadurch aus, dass die Strömungsöffnungen als Spalte zwischen der Membran und der Trägerplatte ausgebildet sind. Damit lassen sich die Strömungsöffnungen besonders nah an der Außenseite der Wandung des Aufnahmekäfigs platzieren, sodass der Nebenfluss mit hoher und gleichförmiger Fließgeschwindigkeit an der Außenseite entlang geführt werden kann. Alternativ können die Strömungsöffnungen auch in Form mehrerer Kreisabschnitte in der Trägerplatte eingebracht sein oder an deren äußerem Umfang durch entsprechende Aussparungen gebildet sein.

Gemäß einer weitergebildeten Ausführung besitzt die Trägerplatte ein oder mehrere Haltemittel zur Befestigung des Zellkulturträgers innerhalb eines Bioreaktors. Beispielsweise können Rastnasen oder dergleichen genutzt werden, um den Zellkulturträger in einem Well einer Mikrotiterplatte zu verankern. Die Trägerplatte ist dafür beispielsweise als kreisförmige Scheibe geformt, deren Abmessungen an die in Mikrotiterplatten vorgesehenen Wells angepasst sind. Ebenso kann der Zellkulturträger in rohrförmigen Gehäusen oder Schlauchmänteln eingesetzt werden.

Vorteilhaft ist es, wenn die Membran in ihrem Randbereich allseits von der Trägerplatte umschlossen ist und mit mehreren umfangseitig verteilt angeordneten Haltestegen an der Trägerplatte befestigt ist. Damit kann die Membran einfach gehalten sowie geschützt sein und gleichzeitig ist die Ausbildung von Haupt- und Nebenfluss gewährleistet.

Gemäß einer bevorzugten Ausführung besteht der vollständige Zellkulturträger aus Polycarbonat. Andere biokompatible Materialien sind ebenso verwendbar.

Die Erfindung betrifft außerdem eine Zellkulturträgeranordnung zur Kultivierung von biologischem Zellmaterial. Die Zellkulturträgeranordnung umfasst zusätzlich zu dem genannten Zellkulturträger ein Gehäuse, welches einen Strömungsbereich für eine Nährstofflösung bereitstellt. Die Trägerplatte, die Membran und der Aufnahmekäfig sind im Strömungsbereich des Gehäuses positioniert. Innerhalb des Gehäuses sind mehrere Strömungsöffnungen vorhanden, die umfangseitig verteilt außerhalb des Aufnahmekäfigs positioniert sind und die den Nebenfluss der Nährstofflösung gestatten, dessen Fließgeschwindigkeit größer ist als die Fließgeschwindigkeit des durch den Aufnahmekäfig und die Ansiedlungsfläche strömenden Hauptflusses der Nährstofflösung.

Weitere Vorteile und Einzelheiten der Erfindung ergeben sich aus der nachfolgenden Beschreibung einer bevorzugten Ausführungsform, unter Bezugnahme auf die Zeichnung. Es zeigen:
- Fig. 1: eine Draufsicht auf einen erfindungsgemäßen Zellkulturträger;
- Fig. 2: eine Seitenansicht des Zellkulturträgers gemäß Fig. 1;
- Fig. 3: eine perspektivische Schnittansicht des Zellkulturträgers gemäß den Figuren 1 und 2.

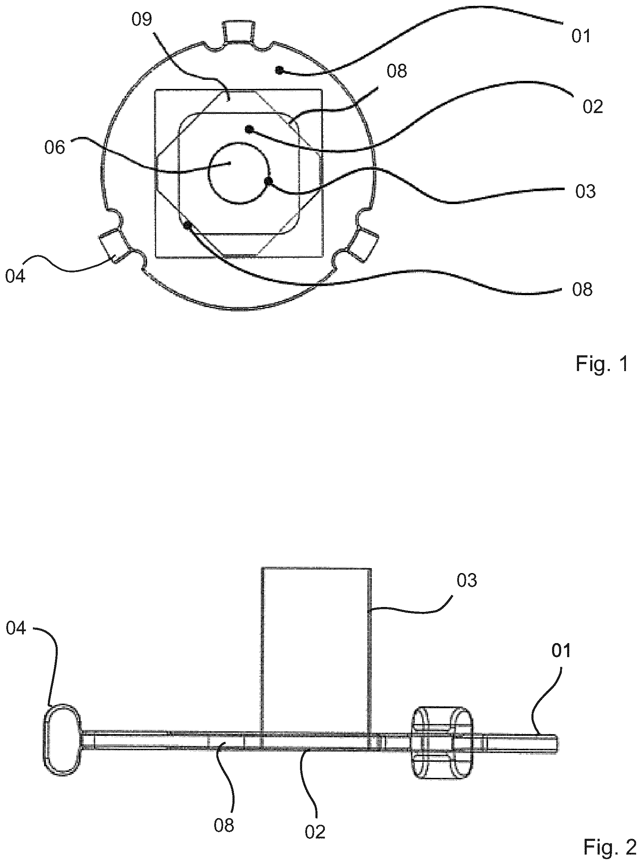

Eine beispielhafte Ausführungsform eines erfindungsgemäßen Zellkulturträgers ist in den Figuren 1 bis 3 dargestellt. Der Zellkulturträger besitzt eine Trägerplatte 01, eine poröse Membran 02 und einen Aufnahmekäfig 03.

Die Trägerplatte 01 ist hier als kreisförmige Scheibe aus Polycarbonat mit einem Durchmesser von ca. 15 mm ausgelegt und weist an ihrem äußeren Rand Haltemittel 04 auf, mit welcher der gesamte Zellkulturträger in einem Gehäuse (nicht gezeigt) befestigt werden kann. Die Membran 02 ist als Formstruktur ebenfalls aus Polycarbonat erzeugt und an ihren Rändern in der Trägerplatte 01 eingespannt. Im Zentrum der Membran 02 ist eine Ansiedlungsfläche 06 vorgesehen, auf welcher Zellmaterial 07 abgelegt werden kann. Mindestens im Bereich der Ansiedlungsfläche 06 ist die Membran porös ausgebildet, sodass einen Nährstofflösung durch die Poren strömen kann.

Der zylindrisch geformte Aufnahmekäfig 03 steht im Bereich der Ansiedlungsfläche 06 auf der Membran 02 auf und ist an dieser befestigt. In anderen Ausführungen kann der Aufnahmekäfig in die Membran eingesetzt sein oder einstückig mit dieser ausgebildet sein. Der Aufnahmekäfig 03 kann ebenfalls aus Polycarbonat hergestellt sein und besitzt beispielsweise einen Durchmesser von 3 mm und eine Höhe von 5 mm. Die Seitenwände des Aufnahmekäfigs 03 haben eine Dicke von ca. 50 µm und sind ebenfalls durchlässig, vorzugsweise porös, sodass auch dort Nährstofflösung bzw. deren Bestandteile durchdringen können. Die obere Stirnseite des Aufnahmekäfigs 03 ist offen, um von dort aus das Zellmaterial 07 einsetzen und auch wieder entnehmen zu können.

In den Eckbereichen der Membran 02 sind Freistellungen vorgesehen, sodass mehrere Strömungsöffnungen 08 in der Trägerplatte 01 verbleiben. Die Strömungsöffnungen 08 sind umfangseitig verteilt außerhalb des Aufnahmekäfigs 03 angeordnet. Zwischen der Membran 02 und der Trägerplatte 01 verbleiben Haltestege 09 zur Halterung der Membran. In abweichenden Ausführungen können die Strömungsöffnungen durch einen mehrteiligen umlaufenden Ringspalt gebildet sein.

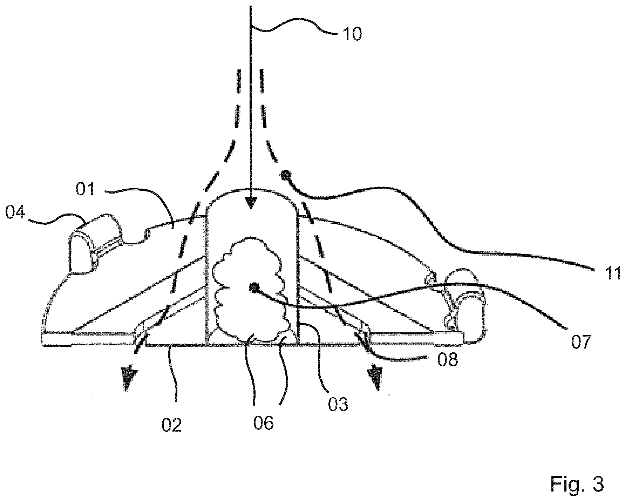

Aus Fig. 3 ist der durch die beschriebene Konstruktion erzeugte Strömungsverlauf für die Nährstofflösung durch Strömungspfeile eingezeichnet. Ein Hauptfluss 10 durchströmt unmittelbar den Innenraum des Aufnahmekäfigs 03, perfundiert dabei das Zellmaterial 07 und tritt aus der Membran 02 wieder aus. Die Porengröße in der Ansiedlungsfläche 06 und der Druck des Hauptflusses 10 sind so gewählt, dass die Fließgeschwindigkeit nicht zu hoch ist, sodass das Zellmaterial 07 nicht durch auftretende Scherkräfte beschädigt wird. Ein Nebenfluss 11 wird außen am Aufnahmekäfig 03 vorbei geführt und fließt über die Strömungsöffnungen 08 ab. Da der Nebenfluss nicht unmittelbar auf das Zellmaterial 07 einwirkt, kann dessen Fließgeschwindigkeit wesentlich höher gewählt werden. Aufgrund der höheren Fließgeschwindigkeit entsteht ein hoher Gradient der Nährstoffe bzw. der in der Nährstofflösung gelösten Gase, zwischen dem Innenbereich des Aufnahmekäfigs 03 und seiner Außenseite, sodass ein Transport durch die poröse Wandung des Aufnahmekäfigs 03 erfolgt. Die Fließgeschwindigkeit des Nebenflusses 11 kann beispielsweise durch die geeignete Wahl des von den Strömungsöffnungen 08 bereitgestellten Querschnitts bestimmt werden. Der Querschnitt der Strömungsöffnungen 08 ist in der Summe regelmäßig größer als die Summe des Querschnitts der Poren im Bereich der Ansiedlungsfläche 06.

### Bezugszeichen

- 01: Trägerplatte
- 02: Membran
- 03: Aufnahmekäfig
- 04: Haltemittel
- 05: -
- 06: Ansiedlungsfläche
- 07: Zellmaterial
- 08: Strömungsöffnungen
- 09: Haltestege
- 10: Hauptfluss
- 11: Nebenfluss

## Patentansprüche

1. Zellkulturträger zur Kultivierung von biologischem Zellmaterial (07) umfassend:
- eine Trägerplatte (01);
- eine Membran (02), die von der Trägerplatte (01) getragen wird und eine Ansiedlungsfläche (06) für das Zellmaterial (07) bereitstellt, wobei die Ansiedlungsfläche (06) für einen Hauptfluss (10) einer Nährstofflösung durchlässig ist;
- einen für die Nährstofflösung durchlässigen Aufnahmekäfig (03), der an einer Stirnseite durch die Membran (02) abgedeckt ist, in welchen das Zellmaterial (07) einbringbar ist und der vom Hauptfluss (10) durchströmbar ist;
**dadurch gekennzeichnet, dass** die Trägerplatte (01) mehrere Strömungsöffnungen (08) bereitstellt, die umfangseitig verteilt außerhalb des Aufnahmekäfigs (03) positioniert sind und die einen Nebenfluss (11) der Nährstofflösung gestatten, dessen Fließgeschwindigkeit größer ist als die Fließgeschwindigkeit des durch den Aufnahmekäfig (03) und die Ansiedlungsfläche (06) strömenden Hauptflusses (10) der Nährstofflösung.

2. Zellkulturträger nach Anspruch 1, **dadurch gekennzeichnet, dass** der Aufnahmekäfig (03) aus demselben porösen Material wie die Membran (02) besteht.

3. Zellkulturträger nach Anspruch 2, **dadurch gekennzeichnet, dass** der Aufnahmekäfig (03) als Hülse mit offenen Stirnseiten geformt ist, wobei die eine Stirnseite auf der Membran (02) aufsteht und die gegenüberliegende Stirnseite offen bleibt zum Einbringen des Zellmaterials (07) und zum Einspeisen des Hauptflusses (10) der Nährstofflösung.

4. Zellkulturträger nach einem der Ansprüche 1 bis 3, **dadurch gekennzeichnet, dass** die Strömungsöffnungen (08) als Spalte zwischen der Membran (02) und der Trägerplatte (01) ausgebildet sind.

5. Zellkulturträger nach einem der Ansprüche 1 bis 4, **dadurch gekennzeichnet, dass** die Trägerplatte (01) Haltemittel (04) zur Befestigung innerhalb eines Bioreaktors besitzt.

6. Zellkulturträger nach einem der Ansprüche 1 bis 5, **dadurch gekennzeichnet, dass** die Membran (02) in ihrem Randbereich von der Trägerplatte (01) umschlossen ist und mit mehreren umfangseitig verteilt angeordneten Haltestegen (09) an der Trägerplatte (01) befestigt ist.

7. Zellkulturträger nach einem der Ansprüche 1 bis 6, **dadurch gekennzeichnet, dass** die Trägerplatte (01) als kreisförmige Scheibe geformt ist, deren Abmessungen an die in Mikrotiterplatten vorgesehenen Wells derart angepasst sind, dass der Zellkulturträger in ein solches Well einsetzbar ist.

8. Zellkulturträger nach Anspruch 7, **dadurch gekennzeichnet, dass** die Membran (02) und die Wandung des Aufnahmekäfigs (03) eine Dicke zwischen 25 und 75 µm aufweisen, dass der Aufnahmekäfig (03) eine axiale Länge zwischen 2,5 bis 3,5 mm aufweist, dass die kreisrunde Trägerplatte (01) einen Durchmesser zwischen 4 bis 18 mm aufweist, und dass die Strömungsöffnungen (08) zusammen einen Querschnitt zwischen 0,5 bis 10 mm² aufweisen.

9. Zellkulturträger nach einem der Ansprüche 1 bis 8, **dadurch gekennzeichnet, dass** er vollständig aus Polycarbonat besteht.

10. Zellkulturträgeranordnung zur Kultivierung von biologischem Zellmaterial (07) umfassend:
- ein Gehäuse, welches einen Strömungsbereich für eine Nährstofflösung bereitstellt;
- eine Trägerplatte (01), die im Strömungsbereich des Gehäuses positioniert ist;
- eine Membran (02), die von der Trägerplatte (01) getragen wird und eine Ansiedlungsfläche (06) für das Zellmaterial (07) bereitstellt, wobei die Ansiedlungsfläche (06) für einen Hauptfluss (10) der Nährstofflösung durchlässig ist;
- einen für die Nährstofflösung durchlässigen Aufnahmekäfig (03), der auf der Membran (02) angeordnet ist und in welchen das Zellmaterial (07) einbringbar ist;
**dadurch gekennzeichnet, dass** innerhalb des Gehäuses mehrere Strömungsöffnungen (08) vorhanden sind, die umfangseitig verteilt außerhalb des Aufnahmekäfigs (03) positioniert sind und die einen Nebenfluss (11) der Nährstofflösung gestatten, dessen Fließgeschwindigkeit größer ist als die Fließgeschwindigkeit des durch den Aufnahmekäfig (03) und die Ansiedlungsfläche (06) strömenden Hauptflusses (10) der Nährstofflösung.

## Claims

1. A cell culture carrier for culturing biological cell material (07), comprising:
- a carrier plate (01);
- a membrane (02) which is carried by the carrier plate (01) and provides a colonisation surface (06) for the cell material (07), wherein the colonisation surface (06) is permeable to a primary flow (10) of a nutrient solution;
- a retaining cage (03) which is permeable to the nutrient solution and which is covered by the membrane (02) on an end face, into which the cell material (07) can be introduced and which can be perfused by the primary flow (10);
**characterized in that** the carrier plate (01) provides a plurality of flow openings (08) which are positioned and distributed around the circumference outside the retaining cage (03) and which permit a secondary flow (11) of the nutrient solution the flow rate of which is greater than the flow rate of the primary flow (10) of the nutrient solution flowing through the retaining cage (03) and the colonisation surface (06).

2. The cell culture carrier as claimed in claim 1, **characterized in that** the retaining cage (03) consists of the same porous material as the membrane (02).

3. The cell culture carrier as claimed in claim 2, **characterized in that** the retaining cage (03) is shaped as a sleeve with open end faces, wherein one end face stands on the membrane (02) and the opposite end face remains open for the introduction of the cell material (07) and for feeding in the primary flow (10) of the nutrient solution.

4. The cell culture carrier as claimed in one of claims 1 to 3, **characterized in that** the flow openings (08) are configured as gaps between the membrane (02) and the carrier plate (01).

5. The cell culture carrier as claimed in one of claims 1 to 4, **characterized in that** the carrier plate (01) has holding means (04) for mounting within a bioreactor.

6. The cell culture carrier as claimed in one of claims 1 to 5, **characterized in that** the edge region of the membrane (02) is enclosed by the carrier plate (01) and is mounted on the carrier plate (01) with a plurality of holding webs (09) which are distributed around the circumference.

7. The cell culture carrier as claimed in one of claims 1 to 6, **characterized in that** the carrier plate (01) is shaped as a circular disk the dimensions of which are adapted to the wells provided in microtitre plates in a manner such that the cell culture carrier can be inserted into such a well.

8. The cell culture carrier as claimed in claim 7, **characterized in that** the membrane (02) and the wall of the retaining cage (03) have a thickness of between 25 and 75 pm, **in that** the retaining cage (03) has an axial length of between 2.5 and 3.5 mm, **in that** the circular carrier plate (01) has a diameter of between 4 and 18 mm, and **in that** the flow openings (08) together have a cross section of between 0.5 and 10 mm².

9. The cell culture carrier as claimed in one of claims 1 to 8, **characterized in that** it consists entirely of polycarbonate.

10. A cell culture carrier assembly for cultivating biological cell material (07), comprising:
- a housing which provides a flow region for a nutrient solution;
- a carrier plate (01) which is positioned in the flow region of the housing;
- a membrane (02) which is carried by the carrier plate (01) and which provides a colonisation surface (06) for the cell material (07), wherein the colonisation surface (06) is permeable to a primary flow (10) of the nutrient solution;
- a retaining cage (03) which is permeable to the nutrient solution, which is disposed on the membrane (02) and into which the cell material (07) can be introduced;
**characterized in that** a plurality of flow openings (08) are provided within the housing and are positioned and distributed around the circumference outside the retaining cage (03) and which permit a secondary flow (11) of the nutrient solution the flow rate of which is greater than the flow rate of the primary flow (10) of the nutrient solution flowing through the retaining cage (03) and the colonisation surface (06).

## Revendications

1. Support de culture cellulaire, destiné à cultiver une matière cellulaire (07) biologique, comprenant :
- une plaque de support (01) ;
- une membrane (02), qui est portée par la plaque de support (01) et qui met à disposition une surface de colonisation (06) pour la matière cellulaire (07), la surface de colonisation (06) étant perméable à un flux principal (10) d'une solution nutritionnelle ;
- une cage réceptrice (03) perméable à la solution nutritionnelle, qui sur une face frontale, est recouverte par la membrane, dans laquelle l'on peut introduire la matière cellulaire (07) et qui est susceptible d'être traversée par le flux principal (10) ;
**caractérisé en ce que** la plaque de support (01) met à disposition plusieurs ouvertures d'écoulement (08), qui sont positionnées en distribution sur la face périphérique à l'extérieur de la cage réceptrice (03) et qui admettent un flux secondaire (11) de la solution nutritionnelle, dont la vitesse d'écoulement est supérieure à la vitesse d'écoulement du flux principal (10) de la solution nutritionnelle circulant à travers la cage réceptrice (03) et la surface de colonisation (06).

2. Support de culture cellulaire selon la revendication 1, **caractérisé en ce que** la cage réceptrice (03) est constituée de la même matière poreuse que la membrane (02) .

3. Support de culture cellulaire selon la revendication 2, **caractérisé en ce que** la cage réceptrice (03) est façonnée sous la forme d'un manchon à faces frontales ouvertes, l'une face frontale étant debout sur la membrane (02) et la face frontale opposée restant ouverte, pour l'introduction de la matière cellulaire (07) et pour l'alimentation du flux principal (10) de la solution nutritionnelle.

4. Support de culture cellulaire selon l'une quelconque des revendications 1 à 3, **caractérisé en ce que** les ouvertures d'écoulement (08) sont conçues sous la forme de fentes entre la membrane (02) et la plaque de support (01).

5. Support de culture cellulaire selon l'une quelconque des revendications 1 à 4, **caractérisé en ce que** la plaque de support (01) dispose de moyens de maintien (04) destinés à être fixés à l'intérieur d'un bioréacteur.

6. Support de culture cellulaire selon l'une quelconque des revendications 1 à 5, **caractérisé en ce que** dans sa zone de bordure, la membrane (02) est entourée par la plaque de support (01) et est fixée sur la plaque de support (01) à l'aide de plusieurs barrettes de maintien (09), placées en distribution sur la face périphérique.

7. Support de culture cellulaire selon l'une quelconque des revendications 1 à 6, **caractérisé en ce que** la plaque de support (01) est façonnée sous la forme d'un disque circulaire dont les dimensions sont adaptées aux puits prévus dans des plaques de microtitrage de telle sorte que le support de culture cellulaire soit insérable dans un tel puits.

8. Support de culture cellulaire selon la revendication 7, **caractérisé en ce que** la membrane (02) et la paroi de la cage réceptrice (03) présentent une épaisseur comprise entre 25 et 75 µm, **en ce que** la cage réceptrice (03) présente une longueur axiale comprise entre 2,5 et 3,5 mm, **en ce que** la plaque de support (01) circulaire présente un diamètre compris entre 4 et 18 mm et **en ce que** les ouvertures d'écoulement (08) présentent conjointement un section transversale compris entre 0,5 et 10 mm².

9. Support de culture cellulaire selon l'une quelconque des revendications 1 à 8, **caractérisé en ce qu'**il est entièrement constitué en polycarbonate.

10. Agencement de support de culture cellulaire, destiné à cultiver une matière cellulaire (07) biologique, comprenant :
- un boîtier, lequel met à disposition une zone de circulation pour une solution nutritionnelle ;
- une plaque de support (01), qui est positionnée dans la zone de circulation du boîtier ;
- une membrane (02), qui est portée par la plaque de support (01) et qui met à disposition une surface de colonisation (06) pour la matière cellulaire (07), la surface de colonisation (06) étant perméable au flux principal (10) de la solution nutritionnelle ;
- une cage réceptrice (03) perméable à la solution nutritionnelle, qui sur une face frontale, est recouverte par la membrane (02), dans laquelle l'on peut introduire la matière cellulaire (07) ;
**caractérisé en ce qu'**à l'intérieur du boîtier sont présentes plusieurs ouvertures d'écoulement (08) qui sont positionnées en distribution sur la face périphérique à l'extérieur de la cage réceptrice (03) et qui admettent un flux secondaire (11) de la solution nutritionnelle, dont la vitesse d'écoulement est supérieure à la vitesse d'écoulement du flux principal (10) de la solution nutritionnelle circulant à travers la cage réceptrice (03) et la surface de colonisation (06).
